# EUROPEAN PATENT SPECIFICATION

(11) **EP 1 173 607 B1**
(45) Date of publication and mention of the grant of the patent: **04.08.2004**
(21) Application number: 00922682.0
(22) Date of filing: 28.04.2000
(51) Int. Cl.: C12Q 1/34, C12Q 1/54

(54) **METHOD FOR THE DETERMINATION OF DISACCHARIDASES**
VERFAHREN ZUR BESTIMMUNG VON DISACCHARIDASEN
METHODE DE DETERMINATION DES DISACCHARIDASES

(30) Priority: 30.04.1999 FI 990990
(43) Date of publication of application: 23.01.2002
(73) Proprietor: BIOHIT OYJ, 00880 Helsinki (FI)
(72) Inventor: SIPPONEN, Pentti, FIN-02160 Espoo (FI); SUOVANIEMI, Osmo, FIN-00570 Helsinki (FI); TAMMINEN, Jani, FIN-00700 Helsinki (FI)
(74) Representative: Grew, Eva Regina
(86) International application number: PCT/FI2000/000375
(87) International publication number: WO 2000/066765

(56) References cited:
- EP-A1- 0 072 450
- US-A- 5 183 742
- J.A. SMITH ET AL.: 'Small bowel biopsy for disaccharidase levels: evidence that endoscopic forceps biopsy can replace the Crosby capsule' CLINICA CHIMICA ACTA vol. 183, 1989, pages 317 - 321, XP002933663
- T.H. IQBAL ET AL.: 'Small intestinal lactase status, frequency distribution of enzyme activity and milk intake in a multi-ethnic population' CLINICAL NUTRITION vol. 15, 1996, pages 297 - 302, XP002932301
- X. CASANOVAS ET AL.: 'Influence of fosfomycin on intestinal disscharides in rats' CHEMOTHERAPY vol. 23, no. SUPPL. 1, 1977, pages 223 - 226, XP002933660
- HANS A. BUELLER ET AL.: 'Lactose intolerance' ANNU. REV. MED. vol. 41, 1990, pages 141 - 148, XP002933664

## Description

The present invention relates to a method for the determination of disaccharidases in a biopsy sample from the duodenum, usually in connection with a gastroscopic procedure, of a patient suspected of suffering from a condition of disaccharide intolerance, especially lactose intolerance. The present method can easily be carried out as a rapid "bed-side" diagnostic method.

### Background of the invention

Disaccharide intolerance is defined as the limited ability of the organism to digest disaccharides, typically milk sugar, i.e. lactose, but also e.g. maltose intolerance is known. The intolerance is due to a decrease in the activity or the concentration of the corresponding disaccharide digesting enzyme, i.e. of lactase (β-galactosidase) in the case of lactose intolerance, which enzyme is produced in the mucous membrane of the small intestine, or duodenum. The enzyme breaks down the disaccharide to simpler sugars that can then be absorbed into the bloodstream.

Normally, when lactose reaches the digestive system, the lactase enzyme hydrolyzes it to D-glucose and D-galactose. The liver then converts the galactose into glucose, which enters the bloodstream and raises the person's blood glucose level. If lactose is incompletely broken down, the blood glucose level does not rise, and a diagnosis of lactose intolerance is confirmed. The resulting condition, although not usually dangerous, may be very distressing. While not all persons deficient in lactase have symptoms, those who do are considered to be lactose intolerant. See generally Buller, H.A. and Grand, R.J., "Lactose Intolerance," Ann. Rev. Med., Vol. 41, pp. 141-148 (1990).

Common symptoms include nausea, cramps, bloating, gas, and diarrhea, which begin about 30 minutes to 2 hours after eating or drinking foods containing lactose. The symptoms are due to the unabsorbed lactose which in the small testine binds liquid and speeds up the through-put rate to the large intestine, where the bacteria digest the carbohydrates to short chain fatty acids, lactate, carbon dioxide and hydrogen.The severity of the symptoms varies depending on the amount of lactose each individual can tolerate.

Some causes of lactose intolerance are well known. For instance, certain digestive diseases and injuries to the small intestine can reduce the amount of enzymes produced. In rare cases, children are born without the ability to produce lactase. For most people, though, lactase deficiency is a condition that develops naturally over time. After about the age of two years, the body begins to produce less lactase. However, many people may not experience symptoms until they are much older.

Between 30 and 50 million Americans are lactose intolerant. Certain ethnic and racial populations are more widely affected than others. As many as 75 percent of all African-Americans and Native Americans and 90 percent of Asian-Americans are lactose intolerant. In the southern Europe and the Middle East the percentage is about 60, and among arabs as high as 90. The condition is least common among persons of northern European descent, e.g. in Finland 11 % of the population are lactose intolerant, but in the northern Scandinavia, 60 % of the Lapps are lactose intolerant.

Lactose intolerance is conventionally diagnosed using a lactose tolerance test, a hydrogen breath test, a stool acidity test or galactose determination.

The lactose tolerance test is the most common test used for diagnosing lactose intolerance. A blood sample after fasting is taken from the patient for glucose determination, whereafter the patient is given a lactose drink. New blood samples are taken after 20, 40 and 60 minutes. The test shows hypolactasia if clear stomach symptoms develop after 1 to 2 hours after taking the lactose drink and if the increase in the blood glucose level remains below 1.1 mmol/l from the initial value.

The hydrogen breath test measures the amount of hydrogen in the breath. Normally, no hydrogen is detectable in the breath. However, undigested lactose is fermented in the colon by bacteria, a result of which is the formation of many gases, including hydrogen. The hydrogen formed is absorbed from the intestine and carried by the blood stream to the lungs, and exhaled. The patient is given a lactose containing drink, after which the breath is analyzed at regular intervals. Increased hydrogen concentrations in the breath means improper digestion of lactose. The test can be affected by certain foods, medication and smoking.

The stool acidicity test measures lactic acid and other short chain fatty acids produced by colon bacteria by fermenting undigested lactose, which acids can be determined in the stool sample. Galactose can in a simple test be determined in the urine after administration of lactose, the test requiring a semi-quantitative determination method for galactose.

Methods for the determination of disaccharides are previously known, but analysis of the disaccharidase content of a biopsy sample usually requires several steps. First of all, the sample must be homogenized, after which it is incubated with a substrate (lactose, maltose etc.), and then the desired monosaccharide is analysed chemically. Such methods for estimating disaccharidase activities are known from e.g. Clinica Chimica Acta 1989, 183: 317-322, and Clinical Nutrition, 1996, 15: 297-302. The existing methodology is complex and time-consuming. Therefore, there is a need for a single, rapid and specific method of diagnosing disaccharide intolerance, especially lactose intolerance.

The publication EP 72 450 discloses a lactase activity test for infants in conjunction with diagnosing infants for cystic fibrosis (CF), such CF-infants reportedly having increased disaccharidase activities in the meconium. Accordingly, a thin film of a meconium sample is spread on a test device containing lactose, glucose oxidase, a peroxidatively active agent and a chromogen, and if the sample has lactase activity, an easily visible blue colour develops directly beneath the meconium.

### Summary of the invention

The present invention provides a quick and easy method for the determination of disaccharidase enzyme in a biopsy sample taken from the duodenum of an individual suspected of being disaccharide intolerant, which method comprises the steps of
- contacting the said biopsy sample as such with a substrate medium containing the said disaccharide; and
- determining the presence of a desired monosaccharide in the substrate medium by using an assay system for said monosaccharide.

Further areas of applicability of the present invention will be apparent from the detailed description given hereinafter.

### Detailed description of the invention

According to the present invention, disaccharide intolerance is diagnosed in an individual by detecting a deficiency or reduced activity of the corresponding disaccharide digesting enzyme, disaccharidase, in a biopsy sample taken from the duodenum of the individual where the corresponding enzyme is normally produced.

Although reference is made specifically to lactose as the disaccharide and lactase as the corresponding disaccharide digesting enzyme, it is clear that the description equally well applies to methods for diagnosing also other disaccharide intolerance conditions. Such conditions include maltose intolerance, in which case a deficiency of maltase enzyme will be the object of diagnosis, or saccharose intolerance, in which case the enzyme to be diagnosed is saccharidase.

In short, the method comprises detecting the presence of disaccharidase in a biopsy sample taken from the duodenum of an individual suspected of suffering from a condition of disaccharide intolerance, which method comprises a first step of contacting the biopsy sample as such, in intact form, that is in an unprocessed, such as in an unhomogenized and uncomminuted form, with a substrate medium containing the said disaccharide. Any disaccharidase present in the sample digests the disaccharide in the substrate to monosaccharides. In a subsequent step, the presence of a desired monosaccharide so formed in the substrate medium is determined by using an assay system for said monosaccharide.

When the object of diagnosis is lactose intolerance, and the method thus comprises detecting the possible presence or absence of lactase enzyme activity in the biopsy sample, the disaccharide to be used in the substrate medium is lactose. Lactose is digested by any lactase present in the biopsy sample to glucose and galactose, which can be detected in the substrate medium in a known manner.

Maltose, on the other hand, will be digested by the maltase enzyme to two glucose molecules, and saccharose is digested by saccharidase to glucose and fructose.

The method can be carried out in a simple manner, for example by using a substrate medium which in the same solution contains the substrate for the enzyme, that is lactose, if a lactase enzyme deficiency is to be diagnosed, glucose oxidase (or galactose oxidase) enzyme, a peroxidase enzyme and a chromogenic substance. It is also possible to keep one or more of the reagents separate from the other reagents up until the moment of carrying out the test. One such alternative is to keep the chromogenic substance, and/or the glucose or galactose oxidase enzyme, in a separate solution, or for example absorbed onto a suitable medium, for example a gel matrix, or paper, to be contacted with the remaining reagents at the moment of testing. Other modifications of carrying out the test are also possible, and easily construed by a person skilled in the art.

The disaccharidase enzyme in the biopsy sample introduced into the substrate medium will digest the disaccharide in the substrate medium to glucose, galactose and/or fructose, depending on the type of disaccharide. The glucose (or galactose) oxidase enzyme in the same medium, which preferably is buffered to approximately pH 5-7, then oxidizes the glucose or galactose to oxidation products, liberating hydrogen peroxide (H₂O₂). The peroxidase enzyme catalyzes a reaction where the hydrogen peroxide oxidizes the colourless chromogenic substance to form a coloured or otherwise detectable form.

The colour reaction taking place in the substrate is rapid and detectable at room temperature already after a few minutes. The biopsy sample can be a small, e.g. of the order of 1 mm x 1 mm x 1 mm, taken from the duodenum in connection with a gastroscopic procedure. The sample taken is used as such and there is no need to homogenize or otherwise comminute the sample prior to testing. The colour change can be determined either with the bare eye, or can be read with a suitable apparatus e.g. photometrically, fluorometrically or reflectometrically. The method makes it possible to evaluate also the disaccharidase level in the biopsy sample, i.e. to make a semiquantitative analysis, and thus to evaluate the severity of the intolerance condition. The method is easy and rapid to carry out as a 'bed-side test' and requires no complicated laboratory equipment.

The concentrations of the various reagents in the substrate medium are not critical and can be adjusted to provide for optimal testing conditions. The reaction can be carried out in a suitable vessel at room temperature, or it can be provided in a suitable kit-form, the kit containing all the reagents needed for carrying out the test in a single ready-to-use package.

The invention being thus described, it will be obvious that the same may be varied within the scope of the following claims.

## Claims

1. Method for the determination of a disaccharidase enzyme, which is able to digest a disaccharide into monosaccharides, in a biopsy sample taken from the duodenum of an individual to be tested for disaccharide intolerance, which method comprises the steps of
- contacting the said biopsy sample as such with a substrate medium containing the said disaccharide; and
- determining the presence of a desired monosaccharide in the substrate medium by using an assay system for said monosaccharide.

2. The method according to claim 1, wherein the disaccharidase to be determined in the sample is lactase, maltase, or sucrase.

3. The method according to claim 1, wherein the disaccharide is lactose.

4. The method according to claim 3, wherein the monosaccharide to be determined in the substrate medium is glucose.

5. The method according to claim 1, wherein the substrate medium contains disaccharide, glucose and/or galactose oxidase, a peroxidase enzyme and a chromogenic substance.

6. The method according to claim 4, wherein the glucose assay system is a reagent strip, preferably a dip-and-read reagent strip.

7. The method according to claim 1, wherein the assay system for determining the monosaccharide is photometric, fluorometric or reflectometric.

## Patentansprüche

1. Verfahren zur Bestimmung eines Disaccharidaseenzyms, das ein Disaccharid in Monosaccharide spalten kann, in einer Biopsieprobe, die aus dem Duodenum eines auf Disaccharid-Intoleranz zu testenden Individuums entnommen wurde, wobei das Verfahren die Schritte umfasst
- Inkontaktbringen der Biopsieprobe als solche mit einem Substratmedium, das das Disaccharid enthält; und
- Bestimmen der Anwesenheit eines gewünschten Monosaccharids in dem Substratmedium unter Verwendung eines Testsystems für das Monosaccharid.

2. Verfahren nach Anspruch 1, wobei die zu bestimmende Disaccharidase in der Probe Lactase, Maltase oder Sucrase ist.

3. Verfahren nach Anspruch 1, wobei das Disaccharid Lactose ist.

4. Verfahren nach Anspruch 3, wobei das zu bestimmende Monosaccharid in dem Substratmedium Glucose ist.

5. Verfahren nach Anspruch 1, wobei das Substratmedium ein Disaccharid, Glucose- und/oder Galactoseoxidase, ein Peroxidaseenzym und eine chromogene Substanz enthält.

6. Verfahren nach Anspruch 4, wobei das Glucosetestsystem ein Reagenzstreifen, vorzugsweise ein Reagenzstreifen zum Eintauchen und Ablesen, ist.

7. Verfahren nach Anspruch 1, wobei das Testsystem zur Bestimmung des Monosaccharids photometrisch, fluorometrisch oder reflektometrisch ist.

## Revendications

1. Procédé de mise en évidence d'une disaccharidase enzyme capable de digérer un disaccharide en monosaccharides, dans un prélèvement biopsique réalisé au niveau du duodénum d'un individu chez qui l'on recherche une intolérance aux disaccharides, ledit procédé comprenant les étapes suivantes :
- mise en contact dudit prélèvement biopsique en tant que tel avec un substrat contenant ledit disaccharide ; et
- mise en évidence de la présence dans le substrat du monosaccharide recherché au moyen d'un système de dosage dudit monosaccharide.

2. Procédé selon la revendication 1, dans lequel la disaccharidase devant être mise en évidence dans le prélèvement est la lactase, la maltase ou la sucrase.

3. Procédé selon la revendication 1, dans lequel le disaccharide est le lactose.

4. Procédé selon la revendication 3, dans lequel le monosaccharide devant être mis en évidence dans le substrat est le glucose.

5. Procédé selon la revendication 1, dans lequel le substrat contient une disaccharide, glucose et/ou galactose oxydase, une enzyme peroxydase et une substance chromogénique.

6. Procédé selon la revendication 4, dans lequel le système de dosage du glucose est une bandelette réactive, de préférence une bandelette réactive à lecture immédiate.

7. Procédé selon la revendication 1, dans lequel le système de dosage permettant la mise en évidence du monosaccharide est photométrique, fluorimétrique ou réflectométrique.
